# EUROPÄISCHE PATENTSCHRIFT

(11) **EP 1 468 656 B1**
(45) Veröffentlichungstag und Bekanntmachung des Hinweises auf die Patenterteilung: **05.10.2005**
(21) Anmeldenummer: 04008606.8
(22) Anmeldetag: 08.04.2004
(51) Int. Cl.: A61B 17/80

(54) **Osteosynthese-Vorrichtung**
Osteosynthesis device
Dispositif d'ostéosynthèse

(30) Priorität: 17.04.2003 DE 10317871
(43) Veröffentlichungstag der Anmeldung: 20.10.2004
(73) Patentinhaber: Stryker Leibinger GmbH & Co. KG, 79111 Freiburg (DE)
(72) Erfinder: Knöpfle, Christian, 78166 Donaueschingen (DE)
(74) Vertreter: Röthinger, Rainer

(56) Entgegenhaltungen:
- EP-A- 0 897 697
- US-A- 5 344 421
- US-A- 5 951 557
- US-A1- 2003 078 583

## Beschreibung

### Gebiet der Erfindung

Die Erfindung betrifft eine Osteosynthese-Vorrichtung mit einem langgestreckten Grundkörper und einem entlang des Grundkörpers verschiebbaren Gleiter. Derartige Osteosynthese-Vorrichtungen werden zur Fixation von Knochenfragmenten und insbesondere von Fragmenten eines Kieferknochens verwendet.

### Hintergrund der Erfindung

Nach Unfällen, wenn ein Knochen in Knochenfragmente zerbrochen ist, oder im Rahmen einer orthognatischen oder maxillofacialen Behandlung zur chirurgischen Korrektur abnormer Stellungen werden funktionale Fixationsvorrichtungen benötigt. Derartige Fixationsvorrichtungen können einen langgestreckten Grundkörper und ein bezüglich des Grundkörpers bewegliches Element umfassen. Sowohl der Grundkörper als auch das bewegliche Element besitzen in der Regel Durchgangsöffnungen für Befestigungselemente wie Knochenschrauben.

Bei einem chirurgischen Eingriff wird in einem ersten Schritt ein Ende des Grundkörpers an einem ersten Knochenfragment befestigt und das bewegliche Element mit dem anderen Knochenfragment verbunden. Das bewegliche Element hat die Aufgabe, vor einer endgültigen Fixation der einzelnen Knochenfragmente mittels des Grundkörpers eine gewisse Beweglichkeit der zu fixierenden Knochenfragmente relativ zueinander zu ermöglichen. Anschließend kann ein genaues Positionieren der zu fixierenden Knochenfragmente erfolgen. Nach dem Positionieren wird das noch nicht befestigte Ende des Grundkörpers an dem zweiten Knochenfragment befestigt und das bewegliche Element von der Osteosynthese-Vorrichtung gelöst. Derartige Osteosynthese-Vorrichtungen mit abnehmbarem beweglichen Element sind beispielsweise aus der WO 97/01991 A1 und der WO 98/44849 A2 bekannt.

In der WO 97/01991 A1 wird eine Osteosynthese-Vorrichtung mit einem langgestreckten Grundkörper beschrieben, der an seinen gegenüberliegenden Enden jeweils zwei Durchgangsöffnungen für ein Befestigungselement besitzt und der dazwischen ein Langloch aufweist. Die Osteosynthese-Vorrichtung umfasst ferner einen in Längsrichtung des Grundkörpers verschiebbaren, abnehmbaren Gleiter, der mit einem sich quer zum Langloch des Grundkörpers erstreckenden Langloch versehen ist. Die Kombination aus den beiden senkrecht zueinander verlaufenden und sich überlappenden Langlöchern gestattet nach dem Befestigen eines Endes des Grundkörpers sowie des Gleiters an den zu fixierenden Knochenfragmenten eine weitestgehend freie Positionierbarkeit der Knochenfragmente relativ zueinander.

Die in der WO 98/44849 A2 (Basis für den Oberbegriff des Anspruchs 1) beschriebene Osteosynthese-Vorrichtung besitzt ebenfalls einen langgestreckten Grundkörper mit einer Mehrzahl von an gegenüberliegenden Enden des Grundkörpers angeordneten Durchgangsöffnungen für Befestigungselemente sowie mit einem zentralen Langloch. Anstelle eines Gleiters ist diese Osteosynthese-Vorrichtung mit einem temporär den Grundkörper aufzusetzenden Feststeller versehen. Der Feststeller umfasst eine die Knochenplatte übergreifende Scheibe und eine nicht lösbar mit der Scheibe verbundene Feststellschraube.

Der Erfindung liegt die Aufgabe zugrunde, eine Osteosynthese-Vorrichtung mit einem langgestreckten Grundkörper und einem entlang des Grundkörpers verschiebbaren Gleiter anzugeben, die sowohl beim provisorischen Befestigen als auch beim sich daran anschließenden Positionieren der zu fixierenden Knochenfragmente eine gute Handhabbarkeit besitzt.

### Zusammenfassung der Erfindung

Diese Aufgabe wird erfindungsgemäß gelöst durch eine Osteosynthese-Vorrichtung mit einem langgestreckten Grundkörper, der an seinen gegenüberliegenden Enden jeweils mindestens eine Durchgangsöffnung für ein Befestigungselement besitzt und der dazwischen ein Langloch aufweist, sowie mit einem entlang des Grundkörpers verschiebbaren Gleiter, der im Bereich des Langlochs mit einer Durchgangsöffnung für ein Befestigungselement versehen ist. Die Osteosynthese-Vorrichtung umfasst ferner eine Koppeleinrichtung zum unverlierbaren Koppeln des Gleiters mit dem Grundkörper, wobei eine Führung des mit dem Grundkörper gekoppelten Gleiters sowohl entlang einer Längsachse des Grundkörpers als auch senkrecht zu der Längsachse gewährleistet ist.

Die Koppeleinrichtung kann im Bereich des Grundkörpers oder im Bereich des Gleiters ausgebildet sein. Komponenten der Koppeleinrichtung können zwischen dem Grundkörper und dem Gleiter verteilt sein. Auch ist es möglich, dass die wesentlichen Komponenten der Koppeleinrichtung und des Gleiters übereinstimmen.

Vorzugsweise umfasst die Koppeleinrichtung zwei miteinander verbundene Auflageelemente. Sofern die Koppeleinrichtung im Bereich des Gleiters ausgebildet ist, können diese Auflageelemente mit gegenüberliegenden Oberflächen des Grundkörpers zusammenwirken. Sollte die Koppeleinrichtung im Bereich des Grundkörpers ausgebildet sein, wirken die Auflageelemente zweckmäßigerweise mit gegenüberliegenden Oberflächen des Gleiters zusammen. Der Gleiter kann im Bereich des Langlochs innerhalb des Grundkörpers angeordnet sein.

Die beiden Auflageelemente können mittels mindestens eines Verbindungselements miteinander verbunden sein. Das Verbindungselement oder die Verbindungselemente können innerhalb des Langlochs oder außerhalb des Langlochs angeordnet sein. Auch ist es möglich, Verbindungselemente für die beiden Auflageelemente sowohl innerhalb als auch außerhalb des Langlochs vorzusehen. Die Auflageelemente können an zwei sich gegenüberliegenden lateralen Enden außerhalb des Langlochs (außerhalb oder innerhalb des Grundkörpers) miteinander verbunden sein.

Sofern die beiden Auflageelemente mittels eines sich durch das Langloch erstreckenden Verbindungselements miteinander verbunden sind, kann dieses Verbindungselement einen hohlzylindrischen Abschnitt aufweisen. Der hohlzylindrische Abschnitt kann einen Teil der Durchgangsöffnung des Gleiters bilden.

Die Auflageelemente können eine beliebige Form besitzen. So können die Auflagenelemente beispielsweise eine kreisrunde Gestalt oder eine im wesentlichen rechteckige Gestalt aufweisen.

Zur Begrenzung der Beweglichkeit des Gleiters bezüglich des Grundkörpers kann eine Begrenzungseinrichtung vorhanden sein. Diese Begrenzungseinrichtung kann von dem mindestens einen Verbindungselement, das die Auflageelemente miteinander koppelt, gebildet werden. Es ist jedoch auch möglich, die Begrenzungseinrichtung im Bereich des Grundkörpers vorzusehen. Zweckmäßigerweise begrenzt die Begrenzungseinrichtung die Beweglichkeit des Gleiters derart, dass der Gleiter in keiner seiner Stellungen bezüglich des Grundkörpers lateral über den Grundkörper übersteht.

Der Grundkörper kann auf wenigstens einer seiner Oberflächen einen vertieften Bereich zur Aufnahme eines der Auflageelemente besitzen. Der vertiefte Bereich kann Seitenfläche aufweisen, die eine geschlossene oder offene Kontur definieren. Diese Seitenflächen können beim Zusammenwirken mit dem Gleiter als Begrenzungselemente zur Begrenzung der Beweglichkeit des Gleiters fungieren.

Zweckmäßigerweise ist das Auflageelement derart in dem vertieften Bereich aufgenommen, dass es nicht oder nur unwesentlich über die jeweilige Oberfläche des Grundkörpers übersteht.

Das im Grundkörper ausgebildete Langloch kann eine geschlossene Kontur besitzen oder nach Art eines Schlitzes einseitig offen sein. Zweckmäßigerweise wird das Langloch an seinen Längsseiten von zwei im Wesentlichen parallel zueinander verlaufenden Stegen des Grundkörpers begrenzt. Die geometrischen Dimensionen der Stege können, wie auch die geometrischen Dimensionen der restlichen Abschnitte und das Material des Grundkörpers, derart gewählt sein, dass der Grundkörper über eine gewisse Verformbarkeit verfügt. Dies erleichtert das Anpassen des Grundkörpers an die zu fixierenden Knochenfragmente.

Die Durchgangsöffnung des Gleiters kann als Langloch ausgebildet sein oder einen kreisrunden Querschnitt besitzen. Im Bereich seiner Durchgangsöffnung kann der Gleiter mit einer Vertiefung zur versenkten Aufnahme des Kopfes eines Befestigungselements versehen sein. Die Durchgangsöffnung des Gleiters ist vorteilhafterweise zum lediglich temporären Aufnehmen des Befestigungselements ausgebildet, d.h. das Befestigungselement kann abnehmbar ausgestaltet sein.

Der Grundkörper kann unterschiedliche Gestalt besitzen. So ist es denkbar, dass der Grundkörper im Wesentlichen die Form eines geraden Streifens aufweist. Ein Ende oder beide Enden des Grundkörpers können sich in zwei oder mehr Fortsätze gabeln. An den freien Enden der Fortsätze kann jeweils mindestens eine Durchgangsöffnung für ein Befestigungselement ausgebildet sein.

### Kurze Beschreibung der Zeichnungen

Weitere Vorteile und Einzelheiten der Erfindung ergeben sich aus der nachfolgenden Beschreibung bevorzugter Ausführungsbeispiele und aus den Figuren. Es zeigen:
- Fig. 1: den langgestreckten Grundkörper einer erfindungsgemäßen Osteosynthese-Vorrichtung;
- Fig. 2a-2c: den Gleiter einer erfindungsgemäßen Osteosynthese-Vorrichtung in verschiedenen Ansichten;
- Fig. 3a-3d: eine erfindungsgemäße Osteosynthese-Vorrichtung in verschiedenen Ansichten;
- Fig. 4: die Osteosynthese-Vorrichtung gemäß den Fig. 3a - 3d in einer Ansicht von unten;
- Fig. 5a u. 5b: die Osteosynthese-Vorrichtung gemäß den Fig. 3a-3d mit in Längsrichtung des Grundkörpers verschobenem Gleiter;
- Fig. 6a u. 6b: die Osteosynthese-Vorrichtung gemäß den Fig. 3a-3d mit in Querrichtung des Grundkörpers verschobenem Gleiter; und
- Fig. 7: eine erfindungsgemäße Abwandlung des Gleiters.

### Detaillierte Beschreibung der Ausführungsbeispiele

Nachfolgend wird unter Bezugnahme auf die beigefügten Zeichnungen die erfindungsgemäße Osteosynthese-Vorrichtung anhand zweier Ausführungsbeispiele näher erläutert. Bei diesen Ausführungsbeispielen gelangt eine Koppeleinrichtung zum Einsatz, die bauteilmäßig in wesentlichen Komponenten mit dem Gleiter übereinstimmt. Die Koppeleinrichtung zum unverlierbaren Koppeln des Gleiters mit dem Grundkörper könnte jedoch auch abweichend von diesen Ausführungsbeispielen im Wesentlichen im Bereich des Grundkörpers ausgebildet sein. Außerdem ist darauf hinzuweisen, dass in den folgenden Ausführungsbeispielen die Koppeleinrichtung eine doppelte Funktion erfüllt. Sie gewährleistet einerseits das unverlierbare Koppeln von Gleiter und Grundkörper und fungiert andererseits als Führungseinrichtung, die eine geführte Bewegung des Gleiters sowohl entlang einer Längsachse des Grundkörpers als auch senkrecht dazu ermöglicht. Abweichend hiervon könnten die Funktionen von Koppeleinrichtung einerseits und Führungseinrichtung andererseits mittels separater Einrichtungen realisiert werden.

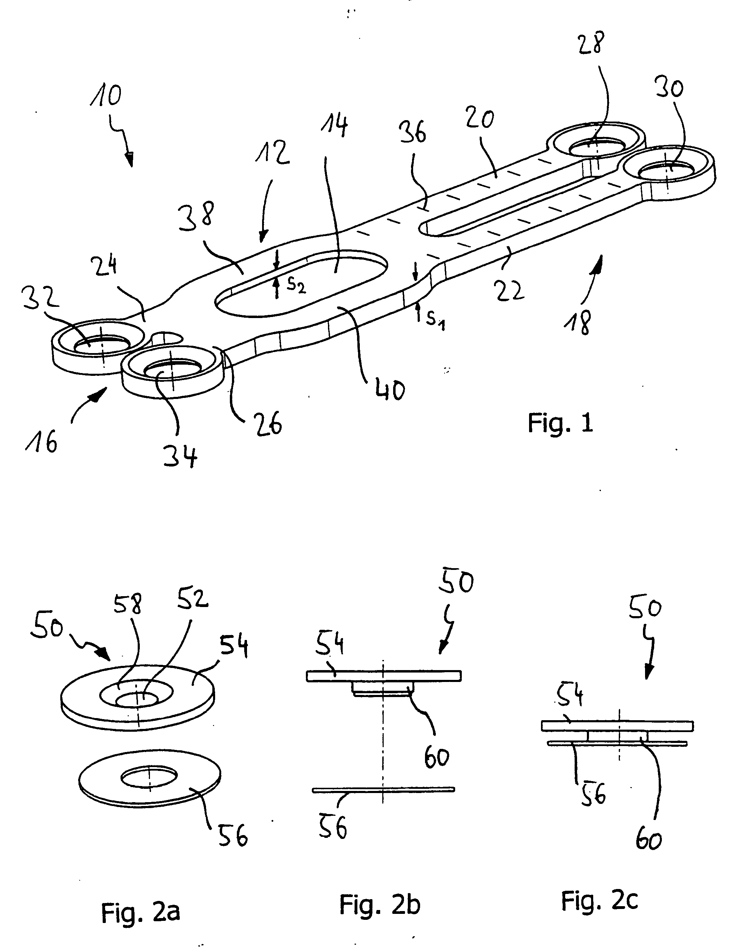

In den Fig. 1 - 6b sind eine erfindungsgemäße Osteosynthese-Vorrichtung gemäß einem ersten Ausführungsbeispiel sowie deren Komponenten dargestellt.

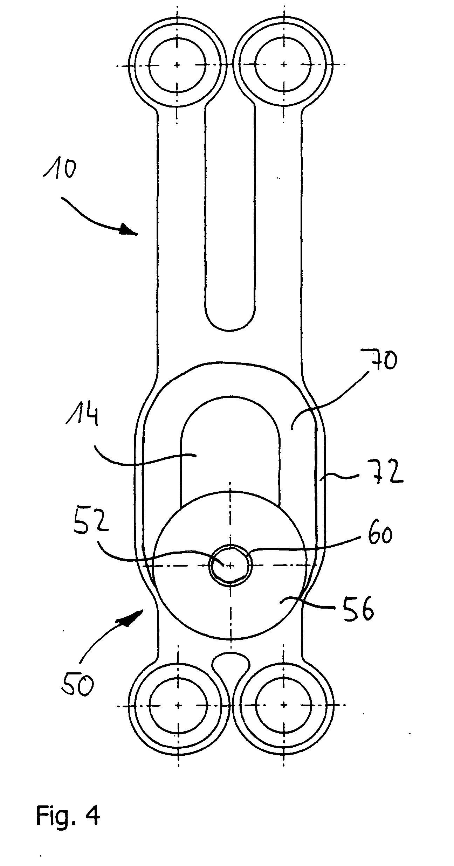

Fig. 1 zeigt den langgestreckten Grundkörper 10 dieser Osteosynthese-Vorrichtung. Der Grundkörper 10 besitzt einen zentralen Abschnitt 12 mit einem Langloch 14. Das Langloch 14 erstreckt sich parallel zu einer in Fig. 1 nicht eingezeichneten Längsachse des Grundkörpers 10. In Fig. 1 ist zu erkennen, dass die Materialstärke s₁ in einem periphären Bereich des zentralen Abschnitts 12 größer ist als die Materialstärke s₂ in einem inneren, an das Langloch 14 angrenzenden Bereich. Dieser Sachverhalt wird weiter unten unter Bezugnahme auf Fig. 4 näher erläutert.

Der zentrale Bereich 12 des Grundkörpers 10 mündet in zwei sich bezüglich des zentralen Abschnitts 12 gegenüberliegende Endabschnitte 16, 18. Der Grundkörper 10 gabelt sich im Bereich jedes der Endabschnitte 16, 18 in je zwei parallel zueinander verlaufende Fortsätze 20., 22, 24, 26. Jeder dieser Fortsätze 20, 22, 24, 26 mündet in je eine Durchgangsöffnung 28, 30, 32, 34 für ein Befestigungselement. Genauer gesagt weisen die Fortsätze 20, 22, 24, 26 an ihren freien Enden kreisringförmige Verbreiterungen auf, deren zentralen Durchbrechungen die Durchgangsöffnungen 28, 30, 32, 34 bilden. Jede dieser Durchgangsöffnungen 28, 30, 32, 34 umfasst eine in der Oberfläche des Grundkörpers 10 ausgebildete, konische Vertiefung. Die konische Vertiefung dient zur versenkten Aufnahme eines Kopfes des in die jeweilige Öffnung 28, 30, 32, 34 eingeführten Befestigungselements.

Die Fortsätze 20, 22 des in Fig. 1 rechten Endabschnitts 18 sind auf ihrer Oberfläche mit einer Reihe von strichförmigen Markierungen 36 versehen. Diese Markierungen sind mittels eines Lasers aufgebracht worden und dienen als Längenskala.

Der Grundkörper 10 ist aus einem biegsamen, biokompatiblen Material wie beispielsweise Titan oder einer Titanlegierung gefertigt. Aufgrund der Gabelung des Grundkörpers im Bereich der Endabschnitte 16, 18 wird die Biegsamkeit der Endabschnitte merklich verbessert. Auch erhöht diese Gabelung die Anpassbarkeit des Grundkörpers 10 an die zu fixierenden Knochenfragmente. Die Biegsamkeit des Grundkörpers 10 ist jedoch nicht auf die Endabschnitte 16, 18 beschränkt. Vielmehr kann der Grundkörper 10 auch im Bereich des Langlochs 14, genauer gesagt im Bereich der das Langloch 14 lateral begrenzenden Stege 38, 40 gebogen werden.

Die erfindungsgemäße Osteosynthese-Vorrichtung gemäß dem ersten Ausführungsbeispiel umfasst neben dem in Fig. 1 dargestellten Grundkörper 10 einen Gleiter 50, der nunmehr unter Bezugnahme auf die Fign. 2a bis 2c näher erläutert wird. Die Fign. 2a und 2b zeigen jeweils eine perspektivische und eine seitliche Explosionsansicht des Gleiters 50. In Fig. 2c ist der Gleiter 50 in einer Seitenansicht in montiertem Zustand dargestellt.

In Fig. 2a deutlich zu erkennen ist die zentrale Durchgangsöffnung 52 für ein Befestigungselement. In auf den Grundkörper montiertem Zustand des Gleiters 50 ist diese Durchgangsöffnung 52 innerhalb des Langlochs des Grundkörpers angeordnet. In den unter Bezugnahme auf die Fign. 1 bis 7 beschriebenen Ausführungsbeispielen fungiert der Gleiter 50 nicht nur als Aufnahme für ein Befestigungselement, sondern gleichzeitig als eine Koppeleinrichtung zum unverlierbaren Koppeln des Gleiters 50 mit dem Grundkörper und besitzt ferner Strukturen, die eine Führung des Gleiters 50 sowohl entlang einer Längsachse des Grundkörpers als auch senkrecht dazu gewährleisten.

Diese Multifunktionalität des Gleiters 50 wird durch die beiden kreisringförmigen Auflageelemente 54, 56 erzielt, die mit gegenüberliegenden Oberflächen des Grundkörpers zusammenwirken. Genauer gesagt wirkt das in den Fign. 2a bis 2c obere Auflageelement 54 mit der in Fig. 1 dargestellten Oberseite des Grundkörpers 10 zusammen und das untere Auflageelement 56 mit dessen den zu fixierenden Knochenfragmenten zugeordneter Unterseite. Das untere Auflageelement 56 ist als eine dünne, kreisringförmige Scheibe ausgebildet. Das obere Auflageelement 54 weist eine größere Stärke auf und besitzt im Bereich der Durchgangsöffnung 52 eine Vertiefung 58 in Gestalt eines sich konisch verengenden Abschnitts. Diese Vertiefung 58 des oberen Auflageelements 54 gestattet eine versenkte Aufnahme des Kopfes eines Befestigungselements und wirkt mit einer konischen Unterseite des Kopfes in selbstzentrierender Weise zusammen.

Die beiden Auflageelemente 54, 56 sind mittels eines hohlzylindrischen Fortsatzes 60 des oberen Auflageelements 54 miteinander verbunden. Das dem unteren Auflageelement 56 zugeordnete Ende des hohlzylindrischen Fortsatzes 60 ist mittels Laserschweißens an dem unteren Auflageelement 56 befestigt. Der hohlzylindrische Fortsatz 60 definiert die Durchgangsöffnung des Gleiters 50.

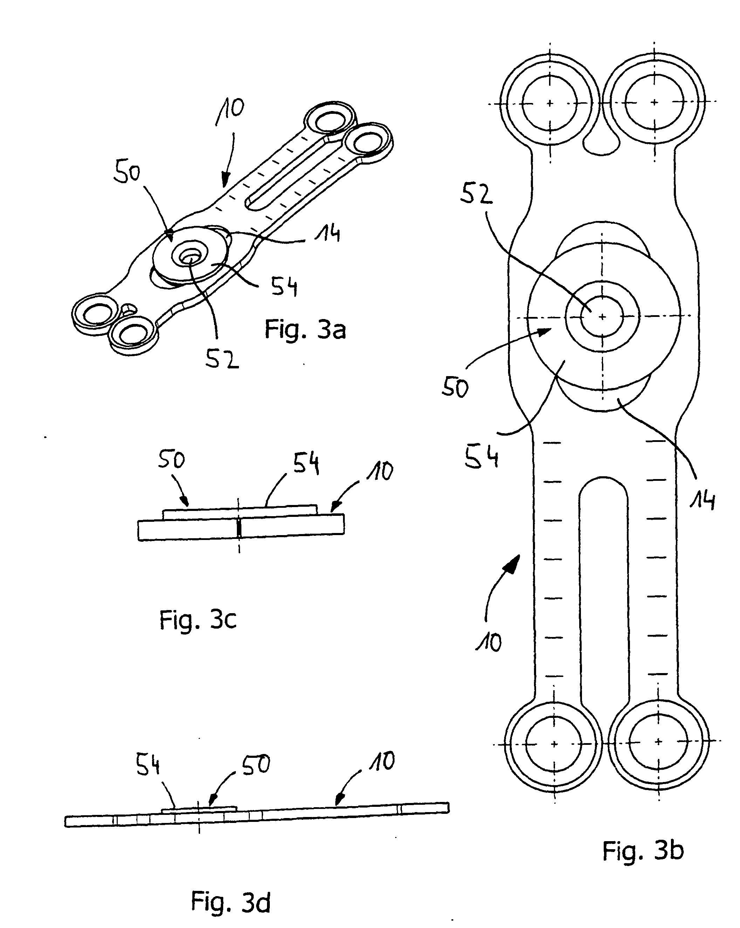

In den Fign. 3a bis 3d ist die erfindungsgemäße Osteosynthese-Vorrichtung gemäß dem ersten Auführungsbeispiel in montiertem Zustand dargestellt. Fig. 3a zeigt eine perspektivische Ansicht, Fig. 3b eine Aufsicht, Fig. 3c eine Vorderansicht und Fig. 3d eine Seitenansicht der Osteosynthese-Vorrichtung.

Zur Montage der Osteosynthese-Vorrichtung wird zunächst das obere Auflageelement 54 derart auf der in Fig. 3b dargestellten Oberseite des Grundkörpers 10 positioniert, dass der die Durchgangsöffnung 52 definierende hohlzylindrische Fortsatz (Bezugszeichen 60 in den Fign. 2b und 2c) in das Langloch 14 des Grundkörpers 10 eingreift. Das obere Auflageelement 54 liegt dabei auf der Oberseite des Grundkörpers 10 auf. Anschließend wird das untere Auflageelement (Bezugszeichen 56 in den Fign. 2a bis 2c) von der in Fig. 4 dargestellten Unterseite des Grundkörpers 10 her konzentrisch zum oberen Auflageelement 54 positioniert und mittels Laserschweißens an dessen hohlzylindrischen Fortsatz befestigt. Damit ist der Gleiter 50 unverlierbar mit dem Grundkörper 10 gekoppelt, da die Dimensionen der Auflageelemente 54, 56 größer gewählt sind als die Dimensionen des Langlochs 14. Gleichzeitig gestattet das Zusammenwirken der Auflageelemente 54, 56 mit der Oberseite bzw. der Unterseite des Grundkörpers 10 eine zuverlässige Führung des Gleiters bezüglich des Grundkörpers.

Der Abstand zwischen den beiden Auflageelementen 54, 56 des Gleiters 50 ist derart gewählt, dass ein gewisses Spiel bezüglich den Oberflächen des Grundkörpers 10 besteht, so dass der Gleiter 50 ohne Kraftaufwand in unterschiedlichen Stellungen bezüglich des Grundkörpers 10 positioniert werden kann. Der Außendurchmesser des in den Fign. 2b und 2c dargestellten hohlzylindrischen Fortsatzes 60, der die beiden Auflageelemente 54, 56 miteinander verbindet, ist geringer gewählt als die Dimensionen des Langlochs 14 des Grundkörpers 10 in Längsrichtung und senkrecht zur Längsrichtung des Grundkörpers 10. Dies ermöglicht es, den Gleiter 50 sowohl entlang einer Längsachse des Grundkörpers als auch senkrecht zu dieser Längsachse zu verschieben.

Wie in den Fign. 3c und 3d dargestellt, steht das obere Auflageelement 54 des Gleiters 50 über die Oberseite des Grundkörpers 10 hervor. Anders verhält es sich auf der Unterseite des Grundkörpers 10. Dort ist kein Überstand des Gleiters 50 (genauer gesagt des unteren Auflageelements) zu erkennen. Das untere Auflageelement ist nämlich in einem vertieften Bereich der Unterseite des Grundkörpers 10 aufgenommen. Dieser Sachverhalt ist in Fig. 4 genauer dargestellt.

Fig. 4 zeigt eine Ansicht der erfindungsgemäßen Osteosynthese-Vorrichtung von unten. Deutlich zu erkennen ist ein angrenzend an das Langloch 14 des Grundkörpers 10 ausgebildeter vertiefter Bereich 70, der von Seitenflächen 72 begrenzt ist. Im Bereich des vertieften Bereichs 70 besitzt der Grundkörper 10 eine geringere Materialstärke s₂ als im Bereich der Seitenflächen 72 (s. Fig. 1, Materialstärken s₁ und s₂). Die Materialstärke des scheibenförmigen unteren Auflageelements 56 ist kleiner oder ungefähr gleich der Differenz s₁ - s₂. Aus diesem Grund ist das Auflagenelement 56 vollständig oder zumindest weitestgehend in dem vertieften Bereich 70 angeordnet und steht, wie in den Fig. 3c und 3d erkennbar, nicht oder nur unwesentlich über die Unterseite des Grundkörpers 10 über. Dies erleichtert die Positionierung der zu fixierenden Knochenfragmente bei nur provisorisch befestigter Osteosynthese-Vorrichtung.

Die Beweglichkeit des Gleiters 50 bezüglich des Grundkörpers 10 wird von den Seitenenden 72 der Vertiefung 70 begrenzt. Mit anderen Worten, die Seitenwände 72 fungieren als Begrenzungseinrichtung hinsichtlich der Beweglichkeit des Gleiters 50.

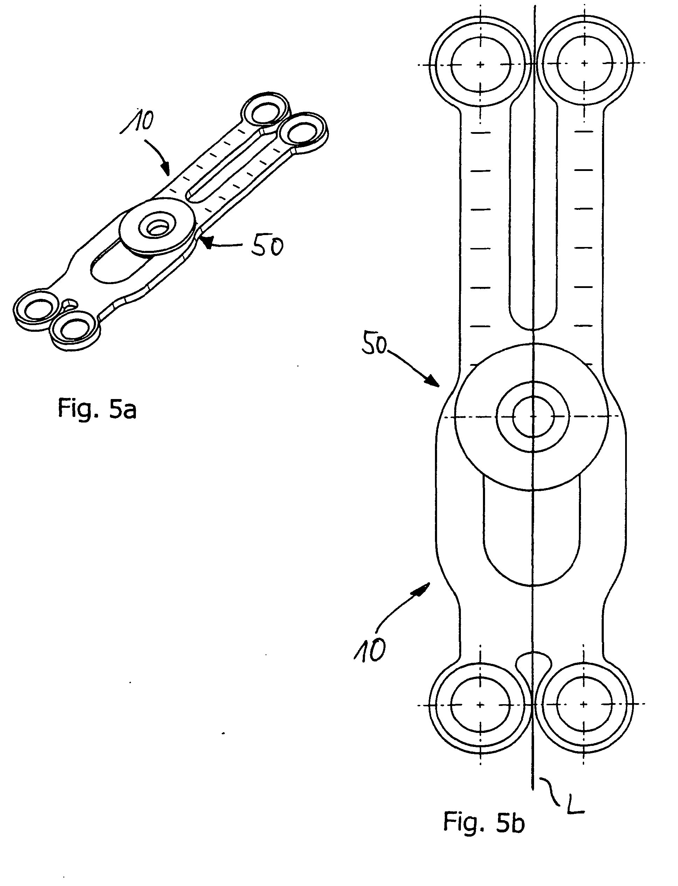

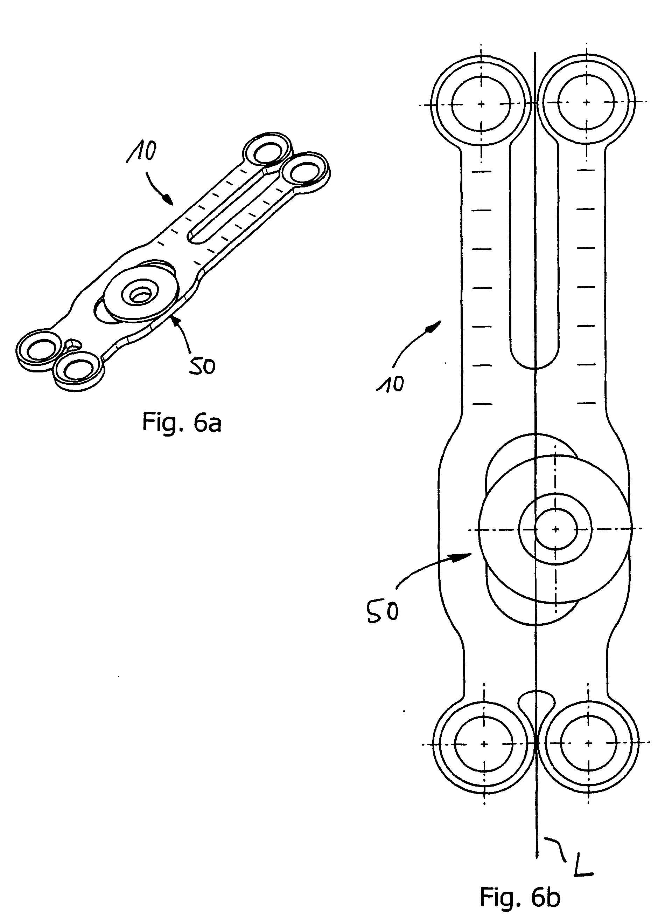

Die Beweglichkeit des Gleiters 50 ist beispielhaft in den Fign. 5a und 5b sowie 6a und 6b dargestellt. Wie diese Figuren zeigen, kann der Gleiter 50 sowohl entlang einer Längsachse L des Grundkörpers 10 als auch senkrecht zu dieser Längsachse L in geführter Weise bewegt werden. Die in Fig. 4 dargestellten, als Begrenzungseinrichtung fungierenden Seitenflächen 72 des vertieften Bereichs 70 gewährleisten, dass der Gleiter 50 in keiner seiner Stellungen bezüglich des Grundkörpers 10 lateral über den Grundkörper 10 übersteht. Dies erleichtert die Handhabung der erfindungsgemäßen Osteosynthese-Vorrichtung.

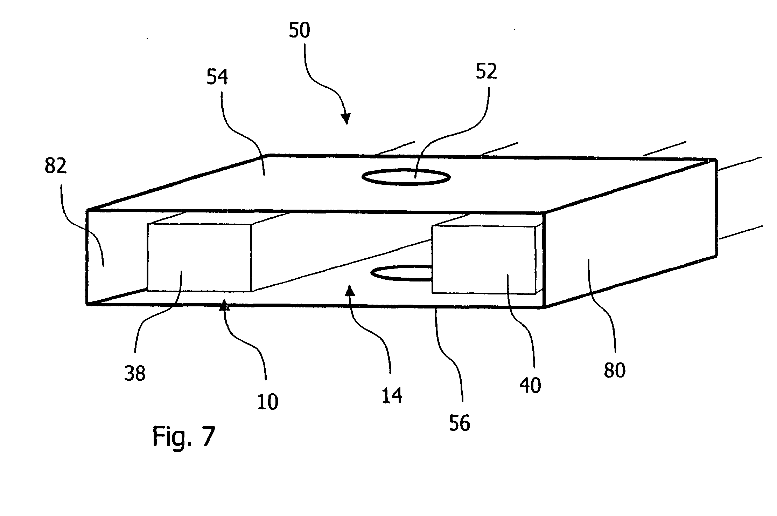

In Fig. 7 ist ausschnittsweise eine Osteosynthese-Vorrichtung gemäß einem zweiten Ausführungsbeispiel der Erfindung gezeigt. Die Osteosynthese-Vorrichtung gemäß dem zweiten Ausführungsbeispiel stimmt in wesentlichen Elementen mit der Osteosynthese-Vorrichtung gemäß dem unter Bezugnahme auf die Fign. 1 bis 6b erläuterten ersten Ausführungsbeispiel überein. Insbesondere ist der Grundkörper 10 der Osteosynthese-Vorrichtung des zweiten Ausführungsbeispiels baugleich mit dem in Fig. 1 dargestellten Grundkörper. Aus diesem Grund werden übereinstimmende Komponenten mit denselben Bezugszeichen wie im ersten Ausführungsbeispiel bezeichnet.

Die maßgeblichen Unterschiede zwischen der Osteosynthese-Vorrichtung gemäß dem ersten Ausführungsbeispiel und der in Fig. 7 ausschnittsweise gezeigten Osteosynthese-Vorrichtung betreffen den Gleiter 50. Bei der Osteosynthese-Vorrichtung gemäß dem zweiten Ausführungsbeispiel sind die Auflageelemente 54, 56 des Gleiters 50 nicht innerhalb des Langlochs 14 miteinander verbunden, sondern mittels zweier Seitenwände 80, 82 außerhalb des Grundkörpers 10. Die Auflageelemente 54, 56 besitzen eine rechteckige Gestalt und erstrecken sich seitlich über den Grundkörper hinweg. Da die Dimensionen der Auflagenelemente 54, 56 senkrecht zur Erstreckung der das Langloch 14 definierenden Stege 38, 40 größer ist als die Breite des Grundkörpers 10 im Bereich dieser Stege 38, 40, ist der Gleiter 50 nicht nur entlang einer Längsachse des Grundkörpers 10, sondern auch senkrecht dazu geführt beweglich.

Bei den Osteosynthese-Vorrichtungen der vorstehend beschriebenen Ausführungsbeispiele besitzt das Langloch des Grundkörpers 10 eine geschlossene Kontur. Das Langloch 14 könnte jedoch, ähnlich dem Langloch des in der WO 98/44849 A2 beschriebenen Grundkörpers nach Art eines Schlitzes einseitig offen ausgestaltet werden.

## Patentansprüche

1. Osteosynthese-Vorrichtung, mit einem langgestreckten Grundkörper (10), der an seinen gegenüberliegenden Enden (16, 18) jeweils mindestens eine Durchgangsöffnung (32, 34; 28, 30) für ein Befestigungselement besitzt und der dazwischen ein Langloch (14) aufweist, sowie mit einem entlang des Grundkörpers (10) verschiebbaren Gleiter (50), der im Bereich des Langlochs (14) mit einer Durchgangsöffnung (52) für ein Befestigungselement versehen ist, **dadurch gekennzeichnet, dass** eine Koppeleinrichtung (54, 56) zum unverlierbaren Koppeln des Gleiters (50) mit dem Grundkörper (10) vorhanden ist, wobei eine Führung des Gleiters (50) sowohl entlang einer Längsachse (L) des Grundkörpers (10) als auch senkrecht dazu gewährleistet ist.

2. Osteosynthese-Vorrichtung nach Anspruch 1, **dadurch gekennzeichnet, dass** die Koppeleinrichtung (54, 56) im Wesentlichen im Bereich des Gleiters (50) ausgebildet ist.

3. Osteosynthese-Vorrichtung nach Anspruch 1 oder 2, **dadurch gekennzeichnet, dass** die Koppeleinrichtung zwei miteinander verbundene Auflageelemente (54, 56) umfasst, die mit gegenüberliegenden Oberflächen des Grundkörpers (10) zusammenwirken.

4. Osteosynthese-Vorrichtung nach Anspruch 3, **dadurch gekennzeichnet, dass** die beiden Auflageelemente (54, 56) mittels mindestens eines außerhalb des Langlochs (14) angeordneten Verbindungselements (80, 82) miteinander verbunden sind.

5. Osteosynthese-Vorrichtung nach Anspruch 3 oder 4, **dadurch gekennzeichnet, dass** die Auflageelemente (54, 56) an zwei sich gegenüberliegenden seitlichen Enden außerhalb des Langlochs (14) miteinander verbunden sind.

6. Osteosynthese-Vorrichtung nach einem der Ansprüche 3 bis 5, **dadurch gekennzeichnet, dass** die Auflageelemente (54, 56) eine im wesentlichen rechteckige Gestalt besitzen.

7. Osteosynthese-Vorrichtung nach Anspruch 3, **dadurch gekennzeichnet, dass** die beiden Auflageelemente (54, 56) mittels eines sich durch das Langloch (14) erstreckenden Verbindungselements (60) miteinander verbunden sind.

8. Osteosynthese-Vorrichtung nach Anspruch 7, **dadurch gekennzeichnet, dass** das Verbindungselement (60) einen hohlzylindrischen Abschnitt aufweist, der einen Teil der Durchgangsöffnung (52) des Gleiters (50) bildet.

9. Osteosynthese-Vorrichtung nach einem der Ansprüche 7 oder 8, **dadurch gekennzeichnet, dass** die Auflageelemente (54, 56) eine kreisrunde Gestalt besitzen.

10. Osteosynthese-Vorrichtung nach einem der Ansprüche 1 bis 9, **dadurch gekennzeichnet, dass** eine Begrenzungseinrichtung (72) vorhanden ist, welche die Beweglichkeit des Gleiters (50) bezüglich des Grundkörpers (10) begrenzt.

11. Osteosynthese-Vorrichtung nach Anspruch 10, **dadurch gekennzeichnet, dass** die Begrenzungseinrichtung von dem mindestens einen Verbindungselement, welches die beiden Auflageelemente miteinander verbindet, gebildet ist.

12. Osteosynthese-Vorrichtung nach Anspruch 10 oder 11, **dadurch gekennzeichnet, dass** die Begrenzungseinrichtung (72) die Beweglichkeit des Gleiters (50) derart begrenzt, dass der Gleiter (50) in keiner seiner Stellungen bezüglich des Grundkörpers (10) lateral über den Grundkörper (10) übersteht.

13. Osteosynthese-Vorrichtung nach einem der Ansprüche 3 bis 12, **dadurch gekennzeichnet, dass** der Grundkörper (10) auf wenigstens einer seiner Oberflächen einen vertieften Bereich (70) zur zumindest bereichsweisen Aufnahme eines der Auflageelemente (56) besitzt.

14. Osteosynthese-Vorrichtung nach Anspruch 13, **dadurch gekennzeichnet, dass** der vertiefte Bereich (70) Seitenflächen (72) aufweist, die beim Zusammenwirken mit dem Gleiter (50) als Begrenzungseinrichtung (56) fungieren.

15. Osteosynthese-Vorrichtung nach Anspruch 13 oder 14, **dadurch gekennzeichnet, dass** das Auflageelement (56) derart in dem vertieften Bereich (70) aufgenommen ist, dass es nicht oder nicht wesentlich über die jeweilige Oberfläche des Grundkörpers (10) übersteht.

16. Osteosynthese-Vorrichtung nach einem der Ansprüche 1 bis 15, **dadurch gekennzeichnet, dass** das Langloch (14) eine geschlossene Kontur besitzt.

17. Osteosynthese-Vorrichtung nach einem der Ansprüche 1 bis 15, **dadurch gekennzeichnet, dass** das Langloch nach Art eines Schlitzes einseitig offen ist.

18. Osteosynthese-Vorrichtung nach einem der Ansprüche 1 bis 17, **dadurch gekennzeichnet, dass** die Durchgangsöffnung (52) des Gleiters (50) einen kreisrunden Querschnitt besitzt.

19. Osteosynthese-Vorrichtung nach einem der Ansprüche 1 bis 18, **dadurch gekennzeichnet, dass** der Gleiter (50) im Bereich seiner Durchgangsöffnung (52) eine Vertiefung (58) zur versenkten Aufnahme eines Kopfes des Befestigungselementes besitzt.

20. Osteosynthese-Vorrichtung nach einem der Ansprüche 1 bis 19, **dadurch gekennzeichnet, dass** die Durchgangsöffnung (52) des Gleiters (50) zum lediglich temporären Aufnehmen des Befestigungselements ausgebildet ist.

21. Osteosynthese-Vorrichtung nach einem der Ansprüche 1 bis 20, **dadurch gekennzeichnet, dass** mindestens eines der Enden (16, 18) des Grundkörpers (10) sich in zwei oder mehr Fortsätze (20, 22; 24, 26) gabelt, an deren freien Enden jeweils mindestens eine Durchgangsöffnung (28, 30; 32, 34) ausgebildet ist.

## Claims

1. Osteosynthesis device having a lengthwise extended base body (10) which respectively has at least one access opening (32, 34; 28, 30) for a fastening element at its opposite ends (16, 18) and which has an elongate hole (14) between them, and having a slider (50) which can be displaced along the base body (10) and is provided with an access opening (52) for a fastening element in the region of the elongate hole (14), **characterized in that** there is a coupling instrument (54, 56) for non-release coupling of the slider (50) with the base body (10), guiding of the slider (50) being ensured both along a longitudinal axis (L) of the base body (10) and perpendicularly thereto.

2. Osteosynthesis device according to Claim 1, **characterized in that** the coupling instrument (54, 56) is formed essentially in the region of the slider (50).

3. Osteosynthesis device according to Claim 1 or 2, **characterized in that** the coupling instrument comprises two mutually connected bearing elements (54, 56), which interact with opposite surfaces of the base body (10).

4. Osteosynthesis device according to Claim 3, **characterized in that** the two bearing elements (54, 56) are connected to each other by means of at least one connecting element (80, 82) arranged outside the elongate hole (14).

5. Osteosynthesis device according to Claim 3 or 4, **characterized in that** the bearing elements (54, 56) are connected to each other at two mutually opposite lateral ends outside the elongate hole (14).

6. Osteosynthesis device according to one of Claims 3 to 5, **characterized in that** the bearing elements (54, 56) have an essentially rectangular configuration.

7. Osteosynthesis device according to Claim 3, **characterized in that** the two bearing elements (54, 56) are connected to each other by a connecting element (60) extending through the elongate hole (14).

8. Osteosynthesis device according to Claim 7, **characterized in that** the connecting element (60) has a hollow cylindrical section, which forms a part of the access opening (52) of the slider (50).

9. Osteosynthesis device according to Claim 7 or 8, **characterized in that** the bearing elements (54, 56) have a round circular configuration.

10. Osteosynthesis device according to one of Claims 1 to 9, **characterized in that** there is a limiter instrument (72) which limits the mobility of the slider (50) with respect to the base body (10).

11. Osteosynthesis device according to Claim 10, **characterized in that** the limiter instrument is formed by at least one connecting element, which connects the two bearing elements to each other.

12. Osteosynthesis device according to Claim 10 or 11, **characterized in that** the limiter instrument (72) limits the mobility of the slider (50) so that the slider (50) does not protrude laterally beyond the base body (10) in any of its positions with respect to the base body (10).

13. Osteosynthesis device according to one of Claims 3 to 12, **characterized in that** the base body (10) has an indented region (70) on at least one of its surfaces for at least local reception of one of the bearing elements (56).

14. Osteosynthesis device according to Claim 13, **characterized in that** the indented region (70) has side faces (72) which function as a limiter instrument (56) in conjunction with the slider (50).

15. Osteosynthesis device according to Claim 13 or 14, **characterized in that** the bearing element (56) is received in the indented region (70) so that it does not, or does not substantially, protrude beyond the respective surface of the base body (10).

16. Osteosynthesis device according to one of Claims 1 to 15, **characterized in that** the elongate hole (14) has a closed contour.

17. Osteosynthesis device according to one of Claims 1 to 15, **characterized in that** the elongate hole is open on one side in the manner of a slot.

18. Osteosynthesis device according to one of Claims 1 to 17, **characterized in that** the access opening (52) of the slider (50) has a round circular cross section.

19. Osteosynthesis device according to one of Claims 1 to 18, **characterized in that** the slider (50) has an indentation (58) in the region of its access opening (52) for sunk reception of a head of the fastening element.

20. Osteosynthesis device according to one of Claims 1 to 19, **characterized in that** the access opening (52) of the slider (50) is designed for merely temporary reception of the fastening element.

21. Osteosynthesis device according to one of Claims 1 to 20, **characterized in that** at least one of the ends (16, 18) of the base body (10) is forked into two or more extensions (20, 22; 24, 26), on the free ends of which at least one access opening (28, 30; 32, 34) is respectively formed.

## Revendications

1. Dispositif d'ostéosynthèse, avec un corps de base allongé (10), qui sur ses extrémités opposées (16, 18) comporte respectivement au moins un orifice de passage (32, 34 ; 28, 30) pour un élément de fixation et qui est muni entre d'un trou oblong (14), ainsi qu'avec un coulisseau (50) déplaçable le long du corps de base (10), qui dans la région du trou oblong (14) est muni d'un orifice de passage (52) pour un élément de fixation, **caractérisé en ce qu'**il existe un système de couplage (54, 56) pour le couplage imperdable du coulisseau (50) avec le corps de base (10), un guidage du coulisseau étant assuré aussi bien le long d'un axe longitudinal (L) du corps de base (10) que perpendiculairement à ce dernier.

2. Dispositif d'ostéosynthèse selon la revendication 1, **caractérisé en ce que** le système de couplage (54, 56) est essentiellement conçu dans la région du coulisseau (50).

3. Dispositif d'ostéosynthèse selon la revendication 1 ou 2, **caractérisé en ce que** le système de couplage comprend deux éléments d'appui (54, 56) reliés entre eux, qui coopèrent avec des surfaces opposées du corps de base (10).

4. Dispositif d'ostéosynthèse selon la revendication 3, **caractérisé en ce que** les deux éléments d'appui (54, 56) sont reliés entre eux par au moins un élément de liaison (80, 82) disposé à l'extérieur du trou oblong (14).

5. Dispositif d'ostéosynthèse selon la revendication 3 ou 4, **caractérisé en ce que** les éléments d'appui (54, 56) sont reliés entre eux sur deux extrémités latérales du trou oblong (14) qui sont opposées.

6. Dispositif d'ostéosynthèse selon l'une quelconque des revendications 3 à 5, **caractérisé en ce que** les éléments d'appui (54, 56) ont une forme sensiblement rectangulaire.

7. Dispositif d'ostéosynthèse selon la revendication 3, **caractérisé en ce que** les deux éléments d'appui (54, 56) sont reliés entre eux par un élément de liaison (60) qui s'étend à travers le trou oblong (14).

8. Dispositif d'ostéosynthèse selon la revendication 7, **caractérisé en ce que** l'élément de liaison (60) comporte un tronçon cylindrique creux, qui forme une partie de l'orifice de passage (52) du coulisseau (50).

9. Dispositif d'ostéosynthèse selon l'une quelconque des revendications 7 ou 8, **caractérisé en ce que** les éléments d'appui (54, 56) ont une forme circulaire.

10. Dispositif d'ostéosynthèse selon l'une quelconque des revendications 1 à 9, **caractérisé en ce qu'**il existe un système de limitation (72) qui limite la mobilité du coulisseau (50), par rapport au corps de base (10).

11. Dispositif d'ostéosynthèse selon la revendication 10, **caractérisé en ce que** le système de limitation est formé par au moins un élément de liaison qui relie entre eux les deux éléments d'appui.

12. Dispositif d'ostéosynthèse selon la revendication 10 ou 11, **caractérisé en ce que** le système de limitation (72) limite la mobilité du coulisseau (50) de façon à ce que le coulisseau (50) ne dépasse latéralement du corps de base (10) dans aucune de ses positions par rapport au corps de base (10).

13. Dispositif d'ostéosynthèse selon l'une quelconque des revendications 3 à 12, **caractérisé en ce que** le corps de base (10) comporte sur au moins l'une de ses surfaces une région creusée (70) pour la réception au moins par régions de l'un des éléments d'appui (56).

14. Dispositif d'ostéosynthèse selon la revendication 13, **caractérisé en ce que** la région creusée (70) comporte des surfaces latérales (72), qui en coopérant avec le coulisseau (50) font office de système de limitation (56).

15. Dispositif d'ostéosynthèse selon la revendication 13 ou 14, **caractérisé en ce que** l'élément d'appui (56) est réceptionné dans la région creusée (70), de façon à ne pas dépasser ou à ne pas dépasser de façon significative de la surface respective du corps de base (10).

16. Dispositif d'ostéosynthèse selon l'une quelconque des revendications 1 à 15, **caractérisé en ce que** le trou oblong (14) a un contour fermé.

17. Dispositif d'ostéosynthèse selon l'une quelconque des revendications 1 à 15, **caractérisé en ce que** le trou oblong est ouvert d'un côté, à la manière d'une encoche.

18. Dispositif d'ostéosynthèse selon l'une quelconque des revendications précédentes 1 à 17, **caractérisé en ce que** l'orifice de passage (52) du coulisseau (50) a une section transversale circulaire.

19. Dispositif d'ostéosynthèse selon l'une quelconque des revendications 1 à 18, **caractérisé en ce que** dans la région de son orifice de passage (52), le coulisseau (50) comporte un évidement (58) pour noyer une tête de l'élément de fixation.

20. Dispositif d'ostéosynthèse selon l'une quelconque des revendications 1 à 19, **caractérisé en ce que** l'orifice de passage (52) du coulisseau (50) est uniquement conçu pour réceptionner temporairement l'élément de fixation.

21. Dispositif d'ostéosynthèse selon l'une quelconque des revendications 1 à 20, **caractérisé en ce qu'**au moins l'une des extrémités (16, 18) du corps de base (10) se ramifie en un ou en plusieurs prolongements (20, 22 ; 24, 26), dans les extrémités libres desquelles est formé respectivement au moins un orifice de passage (28, 30 ; 32, 34).
